(19) Europäisches Patentamt
European Patent Office
Office européen des brevets

(11) Publication number: **0 539 058 A2**

# (12) EUROPEAN PATENT APPLICATION

(21) Application number: **92309174.8**

(22) Date of filing: **08.10.92**

(51) Int. Cl.$^5$: **C07C 2/58**, C08F 8/36, B01J 31/00

A request for addition has been filed pursuant to Rule 88 EPC. A decision on the request will be taken during the proceedings before the Examining Division (Guidelines for Examination in the EPO, A-V, 2.2).

(30) Priority: **22.10.91 US 781565**

(43) Date of publication of application: **28.04.93 Bulletin 93/17**

(84) Designated Contracting States: **AT BE CH DE DK ES FR GB GR IE IT LI LU MC NL PT SE**

(71) Applicant: **ROHM AND HAAS COMPANY**
**Independence Mall West**
**Philadelphia Pennsylvania 19105(US)**

(72) Inventor: **Lundquist, Eric Gustave**
**143 Longleat Drive**
**North Wales, Pennsylvania 19454(US)**
Inventor: **Naples, John Otto**
**1570 Dreshertown Road**
**Dresher, Pennsylvania 19025(US)**

(74) Representative: **Tanner, James Percival et al**
**ROHM AND HAAS (UK) LTD. European Operations Patent Department Lennig House**
**2 Mason's Avenue**
**Croydon CR9 3NB (GB)**

## (54) Isoalkane-olefin alkylation process and catalysts therefor.

(57) The present invention provides a process for the alkylation of isoalkanes with olefins using sulfonated polymer catalysts, where the catalyst has a moisture content of less than about 1.0%, and a catalyst useful in such process. Preferred catalysts include sulfonated macroporous crosslinked vinylaromatic polymers and sulfonated carbonaceous adsorbent polymers having surface areas greater than about 400 square meters per gram. The process is useful for the synthesis of branched alkanes which may be used in high-octane gasolines, e.g., trimethylpentanes from isobutane and butenes.

EP 0 539 058 A2

Rank Xerox (UK) Business Services
(3.10/3.5x/3.0.1)

This invention is concerned with a process for alkylating isoalkanes with olefins in the presence of sulfonated polymer catalyst, and to catalysts useful in such a process. More particularly, this invention is concerned with an alkylation process wherein the sulfonated polymer catalyst functions without the need for additional catalyst materials.

It is well known that high-octane gasoline components can be prepared by alkylating isoalkanes with olefins in the presence of certain acid catalysts to produce alkylate, i.e., the branched adduct of the isoalkane alkylation. In recent years, with the advent of catalytic converters in automobiles and the required use of non-leaded gasoline, a need has arisen for higher octane number gasolines. Straight-run gasolines obtained by direct distillation of crude oil contain primarily normal alkanes, e.g., *n*-pentane and *n*-hexane, and lightly branched alkanes, e.g., methylpentane and methylhexane, which have relatively low octane numbers. To produce gasoline suitable for use in modern automobiles it is necessary to add components (additives) of higher octane to straight run gasolines; alkylate represents such an additive.

Isoalkane alkylation is the reaction between an olefin and a branched chain alkane which results in a branched chain alkane having a higher molecular weight than the isoalkane used as the initial reactant. Most commercial alkylation processes used by the petroleum industry involve large volumes of concentrated sulfuric acid or hydrofluoric acid catalyst which are insoluble in the hydrocarbon reactants used. Alkylations are performed in tank or tube reactors using mechanical agitation to bring the reactants and catalyst into contact with each other via emulsification; the resultant emulsified mixtures must be broken to isolate the alkylate stream and the acid catalysts must be recovered and processed for reuse. Many methods have been developed for improving the yields and purity of desired alkylate products when strong mineral acid catalysts are used and also for extending catalyst life, e.g., reduction of catalyst consumption. One such improvement involves adding small amounts of solid sulfonated aromatic cation exchange resins to the conventional sulfuric acid catalyst system for alkylation (Van Dyke, US-A-3,116,346). However, this process must use special means to separate the desired product from the sulfuric acid stream and then dispose of the used sulfuric acid waste.

Sulfonated aromatic cation exchange resins are well known and are used as catalysts for a variety of chemical processes, including alcohol dehydration, olefin hydration, esterification, hydrolysis reactions and the like. However, it has been found and generally accepted that these acid resins, when used alone, have little or no activity as catalysts for the alkylation of hydrocarbons, i.e., isoalkanes with olefins. A method which overcomes the lack of catalytic activity of the acid resin when used alone involves the addition of excess boron trifluoride ($BF_3$) to form a resin•$BF_3$ complex (Huang *et al.*, US-A-3,855,342). The presence of water, e.g., up to 20%, in the acid resin is required to effect the desired alkylation. When used alone the acid resins were found to be ineffective, yielding only trace amounts of olefin polymerization products. In addition, this process involves the handling of a gaseous toxic material (boron trifluoride) and the separation of catalyst byproducts from the product stream.

Gates *et al.*, US-A-4,123,379, disclose the formation of solid superacid complexes based on the reaction of aluminum chloride vapours with sulfonic acid groups of aromatic cation exchange resins. This process involves the handling of highly corrosive aluminum chloride and the generation of hydrogen chloride as a byproduct.

Another approach to provide improved catalysts for the alkylation of isoalkanes with olefins involves the use of perfluoro-sulfonic acid cation exchange resins (Nafion resin - "Nafion" is a trademark) on inert porous supports (McClure *et al.*, US-A-4,056,578). Although the perfluoro-sulfonic acid resin catalysts possess greater inherent acid strength compared to conventional sulfonic acid polymer catalysts, they suffer from lower capacity, e.g., less than 1 milliequivalent per gram (meq/g), versus conventional sulfonic acid resins, e.g., 3-5 milliequivalents per gram, and are generally non-porous, requiring the use of additional porous inert support materials to increase contact between the reactants and the catalyst, further reducing the effective concentration of acid sites. In addition, perfluoro-sulfonic acid resins are based on fluorocarbon polymeric materials and are considerably more costly to manufacture than non-fluorine containing sulfonated catalysts.

GB-A-1,023,426 discloses a method for alkylating aromatic materials, such as benzene, with olefins using sulfonated polystyrene which is lightly crosslinked and non-porous. The alkylation of aromatics occurs more readily than that of isoalkanes and thus does not require a catalyst as strongly acidic as that required for the isoalkane alkylation.

The present invention enables many of the disadvantages of previous alkylation processes to be overcome. For example, it avoids the large volumes of sulfuric acid or hydrofluoric acid used in conventional alkylation processes, difficulties in separating waste acid streams from the desired products and problems with waste acid disposal. By practice of the present invention, therefore, there may be provided a process for isoalkane alkylation which eliminates the recovery and waste treatment of mineral

acid catalysts, provides increased yields of alkylate products, eliminates the use of corrosive catalysts and permits easier separation of desired products from the catalyst by using sulfonated crosslinked polymer catalysts which were previously considered unsatisfactory for isoalkane alkylation.

According to the present invention there is provided a process for isoalkane alkylation, which comprises combining isoalkanes with olefins in the presence of a sulfonated crosslinked polymer catalyst having a moisture content of less than about 1.0%, preferably less than about 0.5%, by weight of the polymer catalyst. Preferably, the alkylation process is performed in the liquid phase.

Another aspect of the invention comprises an improved alkylation process wherein little or no isomerization of the olefin reactants and intermediates occurs prior to the alkylation such that desired alkylate products of high structural selectivity, based on olefin reactant, are obtained.

A further aspect of the invention includes performing the alkylation substantially in the absence of additional catalyst materials.

The catalysts used in the present invention are sulfonated crosslinked polymers which have been treated so that their moisture content is less than about 1.0%, preferably less than about 0.5%. Preferred catalyst compositions comprise sulfonated macroporous crosslinked vinylaromatic polymers and sulfonated carbonaceous adsorbent polymers. Particularly preferred catalyst compositions of the present invention are polysulfonated macroporous poly(ethylvinylbenzene/divinylbenzene) polymers and sulfonated carbonaceous adsorbents, based on partially pyrolyzed sulfonated-macroporous poly-(styrene/ethylvinylbenzene/divinylbenzene) polymers, having moisture contents of less than about 1.0% by weight. Any drying conditions may be used which reduce the moisture level in the catalyst to less than about 1.0%; these include heating the catalyst under vacuum, e.g., at 110°C for at least 8 hours.

Among the polymers which may be sulfonated for use in the present invention are crosslinked vinylaromatic polymers, e.g., poly(divinylbenzene), polystyrene and the like. The vinylaromatic polymers useful in the present invention are those in which at least 50% of the units contain a vinylaromatic group. Preferred are vinylaromatic polymers in which at least 90% of the units contain a vinylaromatic group. Especially preferred are vinylaromatic polymers where at least 98% of the units contain a vinylaromatic group.

Vinylaromatic monomers used to produce the polymers include, among others, styrene, $\alpha$-methylstyrene, vinyltoluene, p-methylstyrene, ethylvinylbenzene, vinylnaphthalene, vinylxylenes, divinylbenzene, trivinylbenzene, vinylisopropenylbenzene, diisopropenylbenzene and the like. Especially preferred are styrene and divinylbenzene, the latter usually containing some ethylvinylbenzene. The vinylaromatic monomers may also include their corresponding halogenated derivatives, i.e., containing one or more halogen groups, such as fluorine, chlorine or bromine. Alternatively, halogenated sulfonated polymer catalysts may be produced by reacting the sulfonated crosslinked vinylaromatic polymer with appropriate halogenating agents, e.g., bromine, chlorine, sulfur monochloride, iron chloride, iron bromide, haloamides and the like, resulting in one or more halogen groups per sulfonated vinylaromatic monomer unit being incorporated into the catalyst.

Suitable crosslinkers, e.g., polyvinyl compounds, and combinations of crosslinkers useful for making the sulfonated crosslinked polymers used in the present invention include, among others, divinylbenzene, divinylnaphthalene, *bis*(vinylphenyl)- methane, trivinylbenzene, ($C_1$-$C_2$)alkyldivinylbenzenes substituted with 1 to 4 ($C_1$-$C_2$)alkyl groups and ($C_1$-$C_2$)alkyltrivinylbenzenes substituted with 1 to 3 ($C_1$-$C_2$)alkyl groups. The polyvinyl crosslinker level in the copolymers of the present invention may be from about 2% to about 98% by weight of the copolymer, with the preferred range being from about 3% to about 80% by weight of the copolymer.

In one embodiment of the present invention the sulfonated crosslinked polymer is a sulfonated macroporous crosslinked vinylaromatic polymer prepared by polysulfonating a macroporous poly-(styrene/ethylvinylbenzene/divinylbenzene) polymer containing from about 8% to about 90% divinylbenzene, from about 5% to about 50% ethylvinylbenzene and from about 0% to about 85% styrene by weight of the polymer.

Also among the catalysts suitable for use in the present invention are those prepared by the sulfonation of carbonaceous adsorbents. Preparation of these adsorbents by pyrolysis of synthetic resins, such as crosslinked polystyrene, or of polymers treated with oxidants to increase the yield of carbonaceous product, has been known for some years. Adsorbents obtained from partially pyrolyzed porous precursors, especially those based on crosslinked, stabilized, i.e., treated with fixatives or oxidants to prevent depolymerization, polyvinyl-aromatics represent an improved version of such materials. Neely, US-A-4,040,990, which is hereby incorporated into the present specification by reference, describes controlled partial pyrolysis of macroporous monosulfonated, or otherwise fixated, polystyrene to produce charred beads maintaining the macroporous structure of the precursor polymer but with microporosity created

during heat treatment. Maroldo *et al.*, US-A-4,839,331, which is hereby incorporated into the present specification by reference, describes partial pyrolysis of polysulfonated macroporous precursor resins to produce improved resins compared to those of Neely. The term "carbonaceous adsorbent", as used herein, includes the particles prepared by the processes of Neely or Maroldo *et al.* from sulfonated or polysulfonated macroporous vinylaromatic polymer resins.

As used herein, macroporous vinylaromatic polymers include macroporous or macroreticular copolymers prepared by known methods in the presence of a precipitant, such as those disclosed in Meitzner *et al.*, US-A-4,256,840, and copolymers into which large pores have been introduced by other methods, such as the technique described in US-A-3,122,514. The precipitant may be present in ratios from about 20 parts per 100 parts of monomer, i.e., 20% on monomer, to about 600 parts per 100 parts of monomer, i.e., 600% on monomer, depending on the crosslinking level and precipitant used. Among the precipitants which may be used are ($C_4$-$C_{10}$)alcohols, e.g., *t*-amyl alcohol, 2-ethylhexanol, methylisobutyl carbinol and the like; ($C_6$-$C_8$)alkanes, e.g., heptane, isooctane and the like; and ($C_7$-$C_{10}$)aromatic hydrocarbons, e.g., toluene, xylene and the like.

The sulfonated resins prepared from macroporous copolymers are referred to as macroporous or macroreticular resins.

As used herein, sulfonation includes sulfonation or polysulfonation processes, unless indicated otherwise. Sulfonation refers to a process using concentrated sulfuric acid or chlorosulfonic acid. Polysulfonation refers to a process which is sufficiently vigorous to introduce an average of more than one sulfonic acid group per accessible aromatic nucleus, e.g., using reagents or combinations of reagents selected from sulfuric acid, oleum, sulfur trioxide, chlorosulfonic acid and the like. Such vigorous sulfonation is usually accompanied by the formation of sulfone crosslinks, in addition to sulfonic acid groups, in which sulfonic acid groups bridge between two aromatic nuclei to form the sulfone crosslinks.

Preferred catalyst compositions of the present invention are polysulfonated macroporous poly(ethylvinylbenzene/divinyl- benzene) polymers having moisture contents of less than about 1.0% by weight. Macroporous poly(ethylvinylbenzene/divinylbenzene) polymers of particular interest are those containing from about 50% to about 90% divinylbenzene by weight of the polymer. Particularly preferred are macroporous poly(ethylvinylbenzene/divinylbenzene) polymers containing about 80% divinylbenzene by weight of the polymer.

Other preferred catalyst compositions are sulfonated carbonaceous adsorbents comprising sulfonated partially pyrolyzed sulfonated-macroporous poly(styrene/ethylvinylbenzene/divinyl-benzene) polymers having moisture contents of less than about 1.0% by weight. By sulfonated partially pyrolyzed sulfonated-macroporous poly(styrene/ethylvinylbenzene/divinylbenzene) polymers we mean those macroporous poly(styrene/ethylvinylbenzene/divinylbenzene) polymers which have been first sulfonated, then partially pyrolyzed and finally sulfonated. As used herein, sulfonated refers to sulfonated or polysulfonated materials, unless indicated otherwise. Especially preferred compositions comprise sulfonated partially pyrolyzed polysulfonated-macroporous poly(styrene/ethylvinylbenzene/ divinylbenzene) polymers. Macroporous poly(styrene/ethylvinyl-benzene/divinylbenzene) polymers of particular interest are those containing from about 8% to about 90% divinylbenzene, from about 5% to about 50% ethylvinylbenzene, and from 0% to about 85% styrene by weight of the polymer. One preferred macroporous polymer contains from about 8% to about 25% divinylbenzene; particularly preferred macroporous polymers of this type contain about 20% divinylbenzene by weight. Another preferred macroporous polymer contains from about 50% to about 90% divinylbenzene with little or no styrene being present, i.e., less than about 5% styrene by weight; particulary preferred macroporous polymers of this type contain about 80% divinylbenzene with essentially no styrene being present, i.e., less than about 1%.

Typically, sulfonated crosslinked polymer catalysts of the present invention have relatively large surface areas resulting from the macroporosity of the starting macroporous copolymers. In general the overall surface areas as measured by nitrogen adsorption range from about 20 to about 1500 square meters per gram ($m^2/g$), preferably from about 400 to about 1500 square meters per gram. With regard to sulfonic acid ($-SO_3H$) capacity, the sulfonated crosslinked polymer catalysts typically contain 0.1 to 6 milliequivalents $-SO_3H$ per gram of dry resin, preferably at least 1 milliequivalent per gram. The sulfonic acid capacity may be easily controlled by methods, e.g., reaction time, temperature, sulfonating agent and the like, which are well known to those skilled in the art of sulfonation reactions.

The isoalkane and olefin reactants which may be used in the process of the present invention are any isoalkane or olefin which may be liquified, for example, solubilized in isoalkane or olefin coreactant or melted to produce the liquid state under reaction conditions. Isoalkanes or olefins containing from 2 to about 40 carbon atoms may be used for alkylation provided that reaction conditions are used which maintain the reactants in the liquid state.

The Isoalkanes which are of potential use in the production of hydrocarbon fuels, e.g., gasoline or diesel fuel, and which may be used in the process of the present invention, contain from 4 to 8 carbon atoms. Isoalkanes which are especially useful in the production of high-octane gasoline additives contain from 4 to 6 carbon atoms. Representative of isoalkane reactants are isobutane, 3-methylhexane, 2-methylhexane, 2-methyl-butane (also known as isopentane), 2,3-dimethylbutane, 2,4-dimethylhexane and the like. Particularly preferred are isopentane and isobutane; most preferred is isobutane. Isoalkanes containing from 6 to about 8 carbon atoms would be useful in the production of diesel fuels.

The Olefins which are of potential use in the production of hydrocarbon fuels, e.g., gasoline or diesel fuel, and which may be used in the process of the present invention, contain 2 to 12 carbon atoms. Olefins which are especially useful in the production of high-octane gasoline additives contain from 2 to 5 carbon atoms. Representative of olefin reactants are 2-butene, isobutene, 1-butene, propylene, ethylene, pentenes, hexene, heptene, octene and the like. Particularly preferred are propylene, $(C_4)$olefins and $(C_5)$olefins. Most preferred is a $(C_4)$olefin, e.g., 1-butene, 2-butene or isobutene either alone or in admixture. Olefins containing from 6 to about 10 carbon atoms would be useful in the production of diesel fuels.

Isoalkane and olefin reactants which are most preferred for the production of high-octane gasoline additives are isobutane and $(C_4)$olefins selected from one or more of 1-butene, 2-butene and isobutene.

The molar ratio of isoalkane to olefin is generally between about 2 and about 50, and preferably between about 2 and about 20. The weight ratio of catalyst to total hydrocarbon, i.e., isoalkane plus olefin reactant, is generally between about 0.001 and about 1.0, preferably between about 0.01 and about 0.1.

Conditions under which the catalysts of the present invention may be used to effect isoalkane alkylation will vary depending upon composition of the olefin mixture, composition of the isoalkane mixture, desired alkylate composition, catalyst activity and the like. In general, the alkylation reactions are conducted in stainless steel pressure reactors using catalysts which have been dried and transferred to the reactor under an inert atmosphere or vacuum conditions, i.e., sufficient to maintain the catalyst moisture level below about 1.0%. Isoalkane or isoalkane-olefin mixtures are transferred to the reactor and, if required, pressurized to maintain liquid phase conditions during the reaction.

Alkylation using the sulfonated polymer catalysts of the present invention is carried out at temperatures below those at which the resin decomposes, i.e., generally below about 160 ° C. The temperature, under normal operating conditions, will be within the range of from about 20 ° to about 150 ° C, preferably from about 50 ° to about 100 ° C, for about 1 to 5 hours, preferably 2 to 3 hours, depending on the catalyst and reactant mixture used.

The pressure used should be sufficient to maintain reactants in the liquid state, generally between about $344.75 \times 10^3$ Pa and about 1.03 MPa (about 50 and about 1500 pounds per square inch), and preferably between about $689.5 \times 10^3$ Pa and about 3.45 MPa (about 100 and about 500 pounds per square inch). When high boiling, e.g., boiling point above about 150 ° C, isoalkane or olefin reactants are used, the alkylation may be conducted under conditions as low as atmospheric pressure.

The alkylation operation may be carried out as a batch, semi-batch, continuous or semi-continuous operation. The time of reaction is usually controlled by the nature of the isoalkane and olefin reactants, the ratio of the reactants, and the temperature and pressure conditions.

In the batch or semi-batch mode it is preferable to charge the hydrocarbon reactants to a closed reactor already containing the catalyst. Although the catalyst may first be mixed with the isoalkane or olefin, preliminary contact between the catalyst and the olefin should be minimized to avoid olefin polymerization. The isoalkane and olefin reactants may be charged as separate streams or they may be premixed. Catalysts may be readily reconditioned after use by rinsing in solvents, e.g., dichloromethane and the like, to remove any buildup of oligomer or polymer.

When the alkylation is carried out under continuous or semi-continuous mode, the hydrocarbon reactants are continuously charged to the reactor containing the catalyst. Preferably, the reaction mixture is stirred and maintained at the desired temperature while a reaction product mixture is continuously removed from the reactor and transferred to a vessel where the mixture is fractionated to remove unreacted isoalkane, e.g., distilled overhead and recycled to the reactor. The desired alkylate product cut is usually withdrawn as a bottom stream from the fractionating vessel.

An especially preferred process of the present invention comprises combining $(C_4\text{-}C_8)$isoalkanes with $(C_2\text{-}C_{12})$olefins in the presence of a sulfonated crosslinked polymer selected from the group consisting of sulfonated macroporous vinylaromatic polymers and sulfonated carbonaceous adsorbent polymers, wherein the sulfonated crosslinked polymer has a moisture content of less than about 0.5% by weight and a surface area of from about 400 to about 1500 square meters per gram.

The most preferred sulfonated crosslinked polymer catalysts of the present invention are those which are prepared by the polysulfonation of macroporous vinylaromatic polymer precursors or the sulfonation of

carbonaceous adsorbent polymers based on partially pyrolyzed polysulfonated macroporous vinylaromatic polymer precursors where the macroporous vinylaromatic polymer precursors contain from about 8% to about 90% divinylbenzene, from about 5% to about 50% ethylvinylbenzene, and from about 0% to about 85% styrene by weight of the polymer.

It is thought that isoalkane alkylation occurs according to the route presented in the following reaction sequence (Scheme I):

**Scheme I**

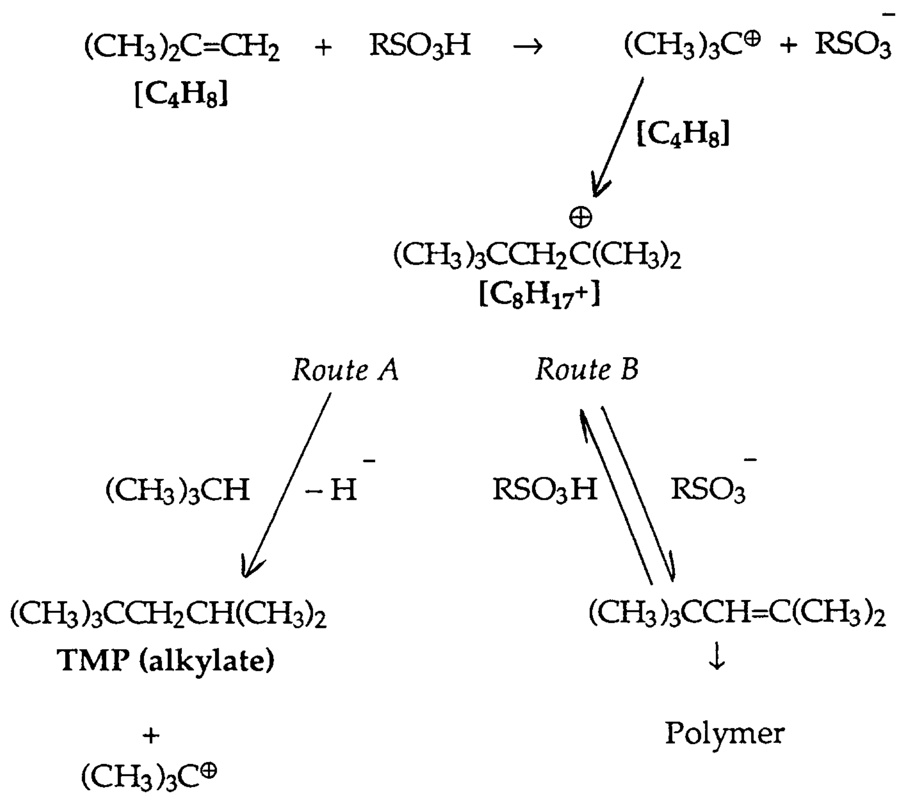


Scheme I uses isobutane ($(CH_3)_3CH$) alkylation with isobutene ($C_4H_8$) to illustrate the competing reactions involved in the presence of catalysts of the present invention. The $C_8H_{17+}$ carbocation is an intermediate in the alkylation scheme and may be generated by most strong acid catalysts. The fate of the $C_8H_{17+}$ carbocation, however, is dependent upon the competition between Route A (hydride transfer → alkylation) and Route B (proton abstraction → olefin → polymer). The acid strength of the catalyst is believed to play a significant role in the fate of the $C_8H_{17+}$ intermediate. The greater the acid strength of the catalyst, $RSO_3H$ (R represents a crosslinked polymeric substrate), the weaker the basicity of the corresponding conjugate base, $RSO_3$ -, resulting in less deprotonation (Route B) of the $C_8H_{17+}$ carbocation relative to hydride transfer (Route A). On the other hand, the weaker the acid used, the greater the basicity of its conjugate base resulting in a greater amount of deprotonation, e.g., olefin or polymer formation, relative to the hydride transfer, e.g., trimethylpentane (TMP) alkylate formation. When conventional strong acids are used as catalysts, e.g., hydrochloric, phosphoric, alkyl or aryl sulfonic and the like, to attempt isoalkane alkylation, the reaction sequence proceeds almost entirely through Route B (olefins, oligomers) without any indication of alkylate product formation (Route A). To be useful as high-octane additives for gasoline, the alkylate product stream should contain a high concentration of branched ($C_8$)alkanes, particularly trimethylpentanes.

One measure of acid strength is based on the well known Hammett $H_0$ acidity scale, where $H_0$ values of about -11 or less, i.e., more negative, are considered representative of super acid activity and $H_0$ values of greater than about -11, i.e., more positive, are representative of strong acids without super acid characteristics, e.g., aqueous mineral acids, arylsulfonic acids and the like. Sulfonated crosslinked polymers of the present invention which are suitable as catalysts for isoalkane alkylation are believed to act as super

acids because of the low moisture levels maintained in the catalyst during use.

The process of the present invention not only successfully effects isoalkane alkylation with olefins, e.g., production of branched ($C_8$)alkanes, but does so while maintaining high structural selectivity based on the nature of the olefin reactant. By this we mean that the identity of the olefin reactant is a dominant factor in determining the isomeric makeup of the alkylate product in which reaction conditions are such that little or no isomerization of the olefin reactants or intermediates occurs prior to alkylation. Prior art processes based on sulfuric add or acid resin•$BF_3$ catalyst systems gave approximately the same alkylate product distribution (TMP content of alkylate) regardless of the ($C_4$)olefin (1-butene, 2-butene or isobutene) used as a reactant (Huang *et al.*, ACS Symposium Series, 55, Industrial and Laboratory Alkylations, pp 75-88(1977)-); this result indicates the formation of common carbocation intermediates due to olefin reactant and/or intermediate isomerization during the alkylation process. As is seen in Examples 10-12 below, the process of the present invention allows selection of the ($C_4$)olefin to be the determining factor in the TMP content of the ($C_8$)alkylate product: 6% TMP from 1-butene, 19% TMP from 2-butene and 56% TMP from isobutene. The catalysts of the present invention, such as those represented by sulfonated macroporous crosslinked vinylaromatic polymers and sulfonated carbonaceous adsorbent polymers having moisture contents less than about 1.0% by weight, maintain high structural selectivity in isoalkane alkylations because they cause the alkylation rate to be significantly greater than any competitive isomerization of the olefin reactants or carbocation intermediates (see Scheme II).

## Scheme II

RSO$_3$H ; olefin ; isoalkane

($C_4$)olefin B ———> ($C_4$)carbocation B ——> ($C_8$)carbocation B ————> alkylate B

$k_{1'}$↑↓ $k_{2'}$↑↓ $k_{3'}$↑↓

RSO$_3$H ; olefin ; isoalkane

($C_4$)olefin A ———> ($C_4$)carbocation A ——> ($C_8$)carbocation A ————> alkylate A

$k_1$ ; $k_2$ ; $k_3$

The reaction path designated $k_{1'}$ involves isomerization of olefin reactants; the reaction paths designated $k_{2'}$ and $k_{3'}$ involve isomerization of olefin intermediates. The reaction path designated $k_1$, $k_2$ and $k_3$ represents the alkylation sequence analogous to Route A in Scheme I. Catalysts of the present invention function in such a way that the alkylation rate ($k_3$) is much greater than any of the competing isomerization reactions ($k_{1'}$, $k_{2'}$, $k_{3'}$). Thus, one is able to control the product composition of the alkylate depending upon the olefin feedstock, whether the desired alkylate product be the highly branched ($C_8$)alkanes preferred as high-octane additives in gasoline or the more linear alkanes, e.g., lightly branched ($C_{11}$-$C_{19}$)alkanes, particulary the ($C_{16}$)alkanes, used in diesel fuel.

Those skilled in the art will appreciate the wide range of conditions which may be used depending on the reactor configuration, the raw materials available and the results desired.

The following Examples are presented to illustrate preferred embodiments of the present invention. All ratios and percentages are by weight, unless indicated otherwise and all reagents are of good commercial quality unless otherwise indicated.

## EXPERIMENTAL

Alkylation experiments were performed in 300-ml SS (316 stainless steel) stirred autoclave reactors. All gases used were of 99.5% purity and were purchased from Matheson. Catalysts were dried 12-16 hours at 110 °C under vacuum [less than about 133.3 Pa (about 1 mm Hg)] unless indicated otherwise.

The following procedure was used: 1-gram (g) of catalyst, dried in a glass autoclave sleeve, was quickly removed from the oven, placed in the reactor and sealed in the reactor while still hot. The reactor was then evacuated [less than about 133.3 Pa (about 1 mm Hg)] for 30 minutes to remove any condensed moisture during the cooling cycle. After returning the cooled catalyst to atmospheric conditions, 100 g of liquified 20/1 molar isobutane/butene mixture [at 1.38 MPa (200 pounds per square inch)] was added. The reactor was then pressurized with nitrogen to 2.07 MPa (300 pounds per square inch) to assure liquid phase conditions throughout the alkylation reaction. The reactor was then heated by electrical means, e.g., heating tape or mantle, to the desired temperature and held there for about 3 hours. The reactor was then cooled to

ambient temperature and the excess gases were vented and collected in a cold trap. The reactor was opened and 10 ml of dichloromethane was introduced to produce a slurry with the hydrocarbon-wetted catalyst. The resultant slurry was filtered and the liquid phase subjected to GC (gas chromatography) analysis. Octane numbers were determined by summing the total of the alkane present and multiplying by the appropriate octane number (olefin products were ignored in this calculation); TMP = trimethylpentanes, DMH = dimethylhexanes, MHep = methylheptanes. Conversion was determined by measuring the amount of hydrocarbon products having 5 or more carbon atoms ($\geqq C_5$) using GC analysis. Selectivity was calculated by dividing the amount of ($\geqq C_5$)alkane by the total amount of hydrocarbon products (alkane + olefin); non-alkane products consisted primarily of ($C_8$)olefins. In the Alkane Product Composition (Alk Prod Comp) description, the amount of hydrocarbon products having more than 9 carbon atoms (% $>C_9$) is composed primarily of oligomers having 12 or more carbon atoms ($\geqq C_{12}$) and includes any ($C_9$)olefins which may be present.

**EXAMPLES 1-9**

Table 1 summarizes data from alkylation reactions conducted at 50° and 100°C at 2.07 MPa (300 pounds per square inch) for 2.5 hours using 20/1 molar isobutane/butene mixture, where the butene mixture contained 50% 2-butene, 25% 1-butene and 25% isobutene. Catalysts A, B, C and D were dried and transferred to the reactors as indicated in the Experimental Section; NMR analysis was used to determine a moisture content of 0.3% by weight under these conditions.

Catalyst A is a sulfonated poly(styrene/ethylvinylbenzene/20% divinylbenzene) macroporous resin with a sulfonic acid capacity of 4.7 meq/g and surface area of 44 $m^2$/g.

Catalyst B is a polysulfonated poly(styrene/ethylvinylbenzene/ 20% divinylbenzene) macroporous resin with a sulfonic acid capacity of 5.4 meq/g and surface area of 44 $m^2$/g.

Catalyst C is a polysulfonated poly(ethylvinylbenzene/80% divinylbenzene) macroporous resin with a sulfonic acid capacity of 4.2 meq/g and surface area of 450 $m^2$/g.

Catalyst D is a sulfonated carbonaceous adsorbent with a sulfonic acid capacity of 2 meq/g and surface area of greater than 1000 $m^2$/g, prepared by sulfonation of partially pyrolyzed polysulfonated-macro-porous poly(styrene/ethylvinylbenzene/20% divinylbenzene), i.e., the macroporous poly-(styrene/ethylvinylbenzene/20% divinylbenzene) polymer was first polysulfonated, then partially pyrolyzed and finally sulfonated with oleum. In this case, the polysulfonated macroporous poly-(styrene/ethylvinylbenzene/20% divinylbenzene) was partially pyrolyzed at 600°C for 3 hours under nitrogen; the partially pyrolyzed resin (50 g) was then sulfonated with 20% oleum (125 g) at 140°C for 3 hours and finally washed and extracted with methanol before being dried for use as an alkylation catalyst.

Catalyst E (comparative) is identical to Catalyst B, except that after being oven-dried it was then allowed to cool at ambient atmospheric conditions before being sealed in the reactor; this catalyst was found to contain 3.8% moisture by NMR analysis.

Examples 1-7 represent catalyst compositions of the present invention while Examples 8 and 9 are presented for comparative purposes since the latter represent sulfonated crosslinked polymer compositions containing more than about 1.0% moisture and are, therefore, ineffective as catalysts for isoalkane alkylation. The 15% and 23% conversions observed for Examples 8 and 9 represent formation of ($C_8$)olefins (Route B in Scheme I) without any indication of alkylate product (Route A in Scheme I); <1% indicates below the limit of detection of the GC analysis used and ND means not determined.

TABLE 1

| Example: | 1 | 2 | 3 | 4 | 5 | 6 | 7 | 8 | 9 |
|---|---|---|---|---|---|---|---|---|---|
| Catalyst: | A | A | B | B | C | C | D | E | E |
| Temp (°C): | 50 | 100 | 50 | 100 | 50 | 100 | 100 | 50 | 100 |
| % Conv: | 30 | 50 | 40 | 58 | 35 | 53 | 35 | 15 | 23 |
| % Select: | 60 | 52 | 70 | 60 | 73 | 65 | 60 | 0 | 0 |
| Alk Prod Comp: | | | | | | | | | |
| % $\leq C_7$ | 18 | 20 | 20 | 22 | 20 | 22 | 18 | <1 | <1 |
| % $C_8$ | 50 | 40 | 52 | 45 | 58 | 47 | 50 | <1 | <1 |
| % $C_9$ | 7 | 8 | 8 | 6 | 7 | 6 | 8 | <1 | <1 |
| % $>C_9$ | 25 | 30 | 20 | 30 | 12 | 20 | 24 | <1 | <1 |
| $C_8$ Composition: | | | | | | | | | |
| % TMP | 10 | 20 | 12 | 23 | 15 | 24 | 22 | <1 | <1 |
| % DMH | 70 | 65 | 70 | 66 | 70 | 67 | 67 | <1 | <1 |
| % MHep | 20 | 15 | 17 | 10 | 15 | 10 | 10 | <1 | <1 |
| Octane #: | 57 | 62 | 62 | 65 | 64 | 65 | 66 | ND | ND |

## EXAMPLES 10-12

Using the same conditions as those described in Example 3, Catalyst B was used to alkylate isobutane using individual pure butenes in place of the butene mixture (used in Examples 1-9) to illustrate the variation of the product mix depending on the specific butenes used (Table 2).

TABLE 2

| Example: | 10 | 11 | 12 |
|---|---|---|---|
| Olefin: | 1-Butene | 2-Butene | Isobutene |
| Alk Prod Comp: | | | |
| % $\leq C_7$ | 10 | 13 | 15 |
| % $C_8$ | 75 | 65 | 53 |
| % $C_9$ | 2 | 6 | 13 |
| % $>C_9$ | 10 | 15 | 25 |
| $C_8$ Composition: | | | |
| % TMP | 6 | 19 | 56 |
| % DMH | 38 | 69 | 31 |
| % MHep | 52 | 12 | 9 |
| Octane #: | 51 | 74 | 82 |

## EXAMPLE 13

A variety of alkylation reactions was conducted at 50°, 80° and 130°C for 3 hours using 5 to 1 volume/volume, of isobutane to butene mixture. The butene mixture was 50% 2-butene, 25% 1-butene and 25% isobutene, by volume. The alkylations were conducted with the following catalysts:

Catalyst F is a sulfonated carbonaceous adsorbent prepared by sulfonation of partially pyrolyzed polysulfonated-macroporous poly(styrene/ethylvinylbenzene/20%divinylbenzene); the macroporous polymer was prepared with methylisobutyl carbinol as a precipitant. In this case, the polysulfonated-macroporous poly(styrene/ethylvinyl-benzene/20% divinylbenzene) was partially pyrolyzed at 400°C for 3 hours under nitrogen and then sulfonated with 20% oleum.

Catalyst G is a sulfonated carbonaceous adsorbent prepared by sulfonation of 20% divinylbenzene); the macroporous polymer was prepared with methylisobutyl carbinol as a precipitant. In this case, the polysulfonated-macroporous poly(styrene/ethylvinyl-benzene/20% divinylbenzene) was partially pyrolyzed at 600°C for 3 hours under nitrogen and then sulfonated with chlorosulfonic acid.

Catalyst H is a sulfonated carbonaceous adsorbent prepared by sulfonation of partially pyrolyzed polysulfonated-macroporous poly-(ethylvinylbenzene/80% divinylbenzene); the macroporous polymer was prepared with toluene as a precipitant. In this case, the polysulfonated-macroporous poly-(ethylvinylbenzene/80% 80% divinyl-benzene) was partially pyrolyzed at 600°C for 3 hours under nitrogen and then sulfonated with chlorosulfonic acid.

Catalyst J is a polysulfonated poly(ethylvinylbenzene/58% divinylbenzene) macroporous resin prepared by sulfonation of the macroporous resin with 20% oleum. In this case, the macroporous resin was made with a mixture of toluene and methylisobutyl carbinol as precipitant.

Catalyst K is a polysulfonated poly(ethylvinylbenzene/58% divinylbenzene) macroporous resin prepared by sulfonation of the macroporous resin with 65% oleum. In this case, the macroporous resin was made with a mixture of toluene and methylisobutyl carbinol as precipitant.

Catalysts F, G, H, J and K were all dried as indicated in the Experimental Section and were found to provide varying degrees of alkylation (as indicated by GC analyses) depending on reaction conditions.

### Claims

**1.** A process for alkylation of isoalkanes with olefins, which comprises combining isoalkanes with olefins in the presence of sulfonated crosslinked polymer having a moisture content of less than about 1.0% by weight.

**2.** A process as claimed in Claim 1, wherein the isoalkane is a ($C_4$-$C_8$)isoalkane.

**3.** A process as claimed in Claim 2, wherein the ($C_4$-$C_8$)isoalkane is selected from isobutane and isopentane.

**4.** A process as claimed in Claim 3, wherein the ($C_4$ - $C_8$) isoalkane is isobutane.

**5.** A process as claimed in any preceding claim, wherein the olefin is a ($C_2$ - $C_{12}$) olefin.

**6.** A process as claimed in Claim 5, wherein the ($C_2$-$C_{12}$)olefin is selected from propylene, ($C_4$)olefins and ($C_5$)olefins.

**7.** A process as claimed in Claim 6, wherein the ($C_4$)olefin is selected from 1-butene, 2-butene and isobutene.

**8.** A process as claimed in any preceding claim, wherein the sulfonated crosslinked polymer is selected from sulfonated macroporous crosslinked vinylaromatic polymers and sulfonated carbonaceous adsorbent polymers.

**9.** A process as claimed in Claim 8, wherein the sulfonated macroporous crosslinked vinylaromatic polymer is prepared by polysulfonating a macroporous poly(styrene/ethylvinylbenzene/divinylbenzene) polymer containing from about 8% to about 90% divinylbenzene, from about 5% to about 50% ethylvinylbenzene and from about 0% to about 85% styrene by weight of the polymer.

10. A process as claimed in Claim 8, wherein the sulfonated carbonaceous adsorbent polymer is prepared by sulfonation of partially pyrolyzed sulfonated-macroporous poly(styrene/ethylvinylbenzene/divinylbenzene) polymer containing from about 8% to about 90% divinylbenzene, from about 5% to about 50% ethylvinylbenzene and from about 0% to about 85% styrene by weight of the polymer.

11. A process as claimed in Claim 10, wherein the partially pyrolyzed sulfonated-macroporous poly(styrene/ethylvinylbenzene/divinylbenzene) polymer is a partially pyrolysed polysulfonated-macroporous poly(styrene/ethylvinylbenzene/divinylbenzene) polymer.

12. A process as claimed in any preceding claim, wherein the moisture content is less than about 0.5% by weight of the sulfonated polymer.

13. A process as claimed in any preceding claim, wherein the sulfonated crosslinked polymer has a surface area of from about 20 to about 1500 square meters per gram, preferably from about 400 to about 1500 square meters per gram.

14. A process as claimed in any preceding claim, wherein the sulfonated crosslinked polymer has a sulfonic acid capacity of 0.1 to 6 milliequivalents per gram, preferably at least 1 milliequivalent per gram.

15. A process as claimed in any preceding claim, which further comprises carrying out the alkylation substantially in the absence of additional catalyst materials.

16. A process as claimed in any preceding claim, wherein reaction conditions allow no, or substantially no, isomerization of olefin reactants and intermediates prior to the alkylation.

17. A process as claimed in any preceding claim, which is performed in the liquid phase.

18. A catalyst composition comprising sulfonated crosslinked polymer selected from polysulfonated macroporous poly(ethylvinylbenzene/divinylbenzene) polymer and sulfonated partially pyrolyzed sulfonated-macroporous poly(styrene/ethylvinylbenzene/divinylbenzene) polymer, wherein the sulfonated crosslinked polymer has a moisture content of less than about 1.0% by weight.

19. A catalyst composition as claimed in Claim 18, wherein the sulfonated partially pyrolyzed sulfonated-macroporous poly(styrene/ethylvinylbenzene/divinylbenzene) polymer is sulfonated partially pyrolyzed polysulfonated-macroporous poly(styrene/ethylvinylbenzene/divinylbenzene) polymer

20. A catalyst composition as claimed in Claim 18 or Claim 19, wherein the macroporous poly(styrene/ethylvinylbenzene/divinylbenzene) polymer contains from about 8% to about 90% divinylbenzene, from about 5% to about 50% ethylvinylbenzene and from about 0% to about 85% styrene by weight of the polymer.

21. A catalyst composition as claimed in Claim 20, wherein the macroporous poly(styrene/ethylvinylbenzene/divinylbenzene) polymer contains:-
   (i) about 20% divinylbenzene by weight of the polymer; or
   (ii) about 50% to about 90% divinylbenzene and less than about 5% styrene by weight of the polymer; or
   (iii) about 80% divinylbenzene and less than about 1% styrene by weight of the polymer.

22. A catalyst as claimed in Claim 18, wherein the macroporous poly(ethylvinylbenzene/divinylbenzene) polymer contains from about 50% to about 90% divinylbenzene, for example about 80% divinylbenzene, by weight of the polymer.

23. A catalyst composition as claimed in any of Claims 18 to 22, wherein the sulfonated polymer has a moisture content of less than about 0.5% by weight.

24. A process for preparing a catalyst composition as claimed in any of Claims 18 to 23, which comprises:

(a) polysulfonating a macroporous poly(ethylvinylbenzene/divinylbenzene) polymer; or
(b) sulfonating a partially pyrolysed sulfonated-macroporous poly-(styrene/ethylvinylbenzene/divinylbenzene) polymer.